# EUROPEAN PATENT APPLICATION

(11) **EP 4 257 668 A1**
(43) Date of publication of application: **11.10.2023**
(21) Application number: 21900640.0
(22) Date of filing: 01.12.2021
(51) Int. Cl.: C12M 3/00, C12N 5/077, A61L 27/38

(54) **CELL CULTURE DEVICE FOR PRODUCING CELL SHEET, KIT FOR PRODUCING CELL SHEET AND METHOD FOR PRODUCING CELL SHEET**

(30) Priority: 02.12.2020 JP 2020200029
(71) Applicant: CellSeed Inc., Tokyo 135-0064 (JP)
(72) Inventor: SUGA Daishi, Tokyo 135-0064 (JP); UENO Yuji, Tokyo 135-0064 (JP); KASUYA Yuzo, Tokyo 135-0064 (JP)
(74) Representative: Schön, Christoph
(86) International application number: PCT/JP2021/044106
(87) International publication number: WO 2022/118889

(57) **Abstract**

Provided are a cell culture device for producing a cell sheet to be used in regenerative medicine and drug discovery research, and a kit for producing a cell sheet. Specifically, a cell detachment inhibition region having cell adhesive force stronger than that of a stimulus-responsive region regardless of the presence or absence of stimulation, and inhibiting cell detachment is directly provided over the entire circumference of a cell culture surface, and the stimulus-responsive region having adhesiveness to cells, which is varied due to an external stimulus, is arranged inside the cell detachment inhibition region, thereby forming a culture device for prevention of cell detachment due to mechanical stimulation such as medium exchange. Moreover, with a support for harvesting cell sheet to be used for the harvest of a cell sheet having given features, the cell sheet can be easily harvested.

## Description

### Technical Field

The present invention relates to a support for harvesting cell sheet and a cell culture device, which are used in fields such as regenerative medicine and drug discovery research and used for collecting cells in the form of a sheet through detachment thereof from the device, and a kit for producing a cell sheet, which comprises them. In particular, the present invention relates to a cell culture device and the like, which are used for culturing with a culture device having a relatively large area, from which cells are easily detached during culture, and for production of a cell sheet from cells having a property of being easily detached. The present invention relates to a kit and a cell culture device, which are used for producing a cell sheet, wherein specific cell types include epithelial cells, mesenchymal stem cells, myoblasts, myocardial cells, smooth muscle cells, cartilage cells, osteoblasts, vascular endothelial cells, fibroblasts, renal cells, and hepatocytes.

### Background Art

Regenerative medicine that promotes the self-renewal ability of damaged organs and tissues by transplanting cells which are collected from tissues with normal functions and culturing cells into patients to repair the tissues and restore functions has attracted increasing attention in recent years. Regenerative medicine is a medical treatment that involves transplanting cells cultured outside a patient's body and tissues constructed from the cultured cells into the patient's body to regenerate damaged tissues and organs and restore lost functions. Cell sheets are cells cultured in the form of sheets that are used in regenerative medicine, and are transplanted into patients and used for constructing tissues. In addition, cell sheets play an important role not only in clinical applications but also in basic research such as drug safety evaluation and drug development.

As a treatment using products for regenerative medicine, there is a method of transplanting a cell sheet obtained by culturing cells and forming a sheet as described above. When adherent cells are cultured on a culture device, the cells adhere to the culture device and the cells adhere to each other, and when the cells reach confluence, they form sheet-shaped cell aggregates (cell sheets). The "confluent (or confluence)" refers to a state in which cells cover the culture surface. For example, epithelial cell sheets are used for treatment of extensive burns, osteoarthritis, prevention of esophageal strictures at the excision site after endoscopic submucosal dissection (ESD) for esophageal cancer, and corneal regeneration, mesenchymal stem cells are used for treatment of encephalopathy such as dementia, breast reconstruction, osteoarthritis/traumatic cartilage deficiency, and ischemic heart disease, and myoblasts are used for treatment of ischemic heart disease, through transplantation thereof to sites where cell regeneration is required. Depending on the purpose of treatment, it is necessary to prepare various cells to be used for culture and various sizes of cell sheets.

When cells are transplanted in regenerative medicine, cells in the form of a sheet should be detached from a cell culture device. Examples of a method for detachment of a cell sheet from a cell culture device include a method that involves weakening the bond between the culture surface of a culture device using a proteolytic enzyme such as dispase, and a method using a cell culture device equipped with a culture surface with variable cell adhesive force.

When a cell sheet is harvested using a proteolytic enzyme, not only the bond between the cells and the culture surface of a cell culture device, but also the bond between the cells is weakened, and there is a problem in that the cell sheet is damaged, leading to significantly poor viability of engraftment after transplantation. In order to avoid this problem, a method that involves harvesting cells without disrupting the protein present in the cell membrane with the use of a cell culture device equipped with a culture surface with variable adhesive force to cells is recommended.

An example of a method for harvesting a cell sheet with the use of a culture surface with variable cell adhesive force is a method using a stimulus-responsive polymer. Examples of the type of a stimulus include temperature, pH, ion, light, electric potential, and magnetism, but temperature stimulation using a temperature-responsive polymer can be suitably used, since the stimulation can be easily applied without affecting cells. Patent Literature 1 discloses a culture device that is a cell culture device coated with a polymer having an upper critical melting temperature and a lower critical melting temperature. With the temperature-responsive polymer, the hydrophobic and hydrophilic balances of a surface of the culture device can be controlled by varying the environmental temperature.

In a culture device coated with a temperature-responsive polymer, the polymer surface shrinks due to the elimination of water from the polymer on a surface of the device at a temperature at the time of cell culture, and thus cells can more easily adhere to the surface. On the other hand, when the temperature is at or below a predetermined temperature, a surface of the device becomes hydrophilic, and the scaffold to which the cells adhere is lost, so that the cells are detached from the device. According to the method using a temperature-responsive polymer, the adhesiveness between cells and a device is lost, but the adhesiveness between cells is not lost, so that cells can be collected in the form of a sheet.

Treatment by regenerative medicine, is treatment with epithelial cells, endothelial cells, and stem cells, in which a cell sheet required for each region to be treated is prepared. Depending on the treatment, the type of cells and size to be prepared also differ, and the ease of handling cells during culture also differs accordingly. For example, when used for inhibiting esophageal strictures after endoscopic submucosal dissection (ESD) for esophageal cancer or for regeneration of corneal membrane, periodontal tissue, a cell sheet having a very large area is not required. On the other hand, when used for treatment of extensive burns, etc., a cell sheet having a large area is required. A cell sheet having a large area is more likely to be detached from a culture device during the production process compared with a cell sheet having a small area. Further, there are cell types such as myoblasts having a property of being extremely easily detached. When a cell sheet having a large area is produced, or as cells proliferate and reach confluence, cells will be more easily detached from the surface of a culture device due to mechanical stimulation such as pipetting operation upon the exchange of culture fluids depending on the type of cells.

When cells are collected as a cell sheet, cells are cultured until they reach confluence, but if cells to be collected as a cell sheet are detached when the medium is aspirated, there is a problem in transplantation. Despite the fact that the operation is carried out with due care, if cells are detached from the edge(s) upon medium exchange etc., during the culture period, they cannot be collected as a cell sheet and cannot be transplanted. In particular, when cells are used for treatment of extensive burns, it is necessary to use a culture device having a culture area as large as possible and harvest all the cultured cell sheets because the transplantation area is large. As described above, when the culture area is large, cells are easily detached, so that cell detachment upon medium exchange etc., has been a big problem.

In a method using a culture device equipped with a stimulus-responsive surface, a cell culture vessel in which a stimulus-responsive surface and a surface having no stimulus-responsiveness are arranged on a surface of a device to regulate the cell adhesiveness is disclosed (Patent Literatures 2 to 4). Patent Literature 2 discloses a cell culture device as a culture vessel for stably holding a cell sheet on a culture surface even after stimulation and easily harvesting the cell sheet, wherein a cell adhesion region having cell adhesiveness encloses a stimulus-responsive region. Patent Literature 3 discloses a cell culture device equipped with a stimulus-responsive region, a cell adhesion region, and a non-cell adhesion region and is equipped with a boundary with the non-cell adhesion region at least a portion of the outer circumference of the stimulus-responsive region in order to provide a cell culture device with which the back side that is the side adhering to the cell scaffold (culture device surface) can be easily observed. Patent Literature 4 discloses a method for preparing a cell sheet that can be detached from a substrate at a desired timing without being detached at an unintended timing during the culture operation.

### Citation List

### Patent Literature

Patent Literature 1: Japanese Patent Laid-Open No. H05-192138
Patent Literature 2: Japanese Patent Laid-Open No. 2019-24350
Patent Literature 3: Japanese Patent Laid-Open No. 2012-105608
Patent Literature 4: Japanese Patent Laid-Open No. 2015-70797

### Summary of Invention

### Technical Problem

However, the vessel described in Patent Literature 2 is used for holding a cell sheet on a culture surface by securing a region to which cells can adhere after the cells are detached from the stimulus-responsive region due to stimulation, and for simply collecting cells by detaching the adhesion site through pipetting or the use of tweezers. Specifically, the cell culture vessel described in Patent Literature 2 is configured so that the cell sheet can be easily detached by pipetting or the like while holding a portion of the cell sheet on the surface of the culture device after stimulation. Therefore, it is not assumed that cells would be detached during the culture period, that is, due to mechanical stimulation upon medium exchange etc., before stimulation. Hence, the vessel is not intended to inhibit cell detachment during the cell culture period, which makes it impossible to collect the cells as a cell sheet.

The culture instrument described in Patent Literature 3 is used for observing the back side by detaching cells in some regions in the form of a sheet(s) by applying stimulation. Therefore, it is assumed that the cell adhesion region that is the side of observing the cell surface remains firmly adhered to the culture device. Therefore, cell detachment due to mechanical stimulation during culture has not been taken into consideration.

The method for preparing a cell sheet described in Patent Literature 4 involves forming in advance a region having no stimulus-responsiveness at a position adjacent to a stimulus-responsive region, culturing cells on the surfaces of these regions, and, upon cell sheet detachment, separating the cells on the surface of one region from the cells on the surface of the other region together with the substrates. It is disclosed that this allows cells to be transported without the use of carriers (support for harvesting cell sheet). Since the cell sheet is produced by cutting the cells together with the substrate, a preferred aspect disclosed involves forming a functional region on a part of the surface of the cell adhesive substrate such as a film or a plate, and then fixing the region on the bottom surface of the cell culture vessel with an adhesive for use. Since cutting and separation are required, such an aspect of directly forming a region containing a stimulus-responsive polymer on the bottom surface of a culture vessel is not realistic.

As described in the embodiment, it is an object to provide a culture device that is equipped with a cell detachment inhibition region which prevents cells being detached against mechanical stimulation during operations such as medium exchange and is capable of collecting cells in the form of a cell sheet. Further, upon development of the culture device of the present embodiment, it has been revealed that the device equipped with the cell detachment inhibition region can prevent cells from being detached from the culture device during the culture period, but causes a problem such that a cell sheet cannot be easily harvested. In order to solve this, we devised the material for a support for harvesting cell sheet to be used for detaching cells in the form of a sheet. An object of the present invention is to provide a kit for producing a cell sheet, which is capable of preventing a cell sheet from being detached and easily harvesting the cell sheet.

Moreover, when used in basic research in regenerative medicine and drug discovery research, it has been required to analyze cells cultured under the same conditions by various methods. For example, a cell sheet is transplanted into an animal model, and then the gene expression in the cell sheets cultured under the same conditions is analyzed by PCR or Western blotting, and the morphology of the cells is observed under a microscope. Since cells to be used for regenerative medicine are not usually cell lines but cells derived from tissues such as skin and mucous membranes obtained from patients, there is a possibility that the cell properties may not always be the same in every culture device. Therefore, it may be questioned whether the changes that have occurred are due to cell variations or changes due to treatment with drugs or the like. An object of the present embodiment is to provide a kit for producing a cell sheet and a culture device, which are capable of harvesting a plurality of culture sheets from one culture device. Through analysis of cells cultured in the same culture devices, it is possible to make the culture conditions exactly the same, and to analyze changes more accurately.

### Solution to Problem

The present invention includes the following cell culture device for producing a cell sheet, kit for producing a cell sheet, and method for producing a cell sheet.
(1) A cell culture device, wherein
   a cell detachment inhibition region having cell adhesive force stronger than that of a stimulus-responsive region regardless of the presence or absence of a stimulus and inhibiting cell detachment is provided over the entire circumference of a cell culture surface,
   the stimulus-responsive region having adhesiveness to cells, which is varied by an external stimulus, is arranged inside the cell detachment inhibition region, and the cell detachment inhibition region has cell adhesiveness 3 or more times and 15 or less times stronger than that of the stimulus-responsive region even in a state where no stimulation is applied to the stimulus-responsive region.
(2) The cell culture device according to (1), wherein the container shape of the cell culture device is a petri dish, a flask, or a multi-well plate, and the stimulus-responsive region and the cell detachment inhibition region are directly provided on the cell culture surface of the cell culture device.
(3) The cell culture device according to (1) or (2), which is for the harvesting of a cell sheet using a support for harvesting cell sheet.
(4) The cell culture device according to any one of (1) to (3), wherein the cell culture surface of the cell culture device has an area of 3.5 cm² or more and 300 cm² or less.
(5) The cell culture device according to any one of (1) to (4), wherein the cell detachment inhibition region is a cell adhesive surface in a temperature range of 1°C or higher and 50°C or lower.
(6) The cell culture device according to (5), wherein the cell adhesive surface is a culture surface not responding to an external stimulus.
(7) The cell culture device according to any one of (1) to (6), wherein the external stimulus is light, a temperature, a pH, or a magnetic field.
(8) The cell culture device according to any one of (1) to (7), wherein the stimulus-responsive region is a temperature-responsive surface responding to temperature stimulation, and a surface from which cells are detached at a temperature of 0°C or higher and 32°C or lower.
(9) The cell culture device according to any one of (1) to (8), wherein the cell detachment inhibition region and the stimulus-responsive region are distinct sections on the same plane.
(10) The cell culture device according to any one of (1) to (9), wherein the cell detachment inhibition region has a width of 2 mm or more and 10 mm or less.
(11) The cell culture device according to any one of (1) to (10), wherein the cell detachment inhibition region is provided so as to divide the cell culture surface in addition to the periphery of the cell culture surface.
(12) The cell culture device according to any one of (1) to (11), wherein the cell detachment inhibition region and the stimulus-responsive region are processed so as to be visually distinguishable.
(13) The cell culture device according to any one of (1) to (12), wherein the cell detachment inhibition region and the stimulus-responsive region are optically transparent.
(14) The cell culture device according to any one of (1) to (13), which is for producing a cell sheet of epithelial cells, mesenchymal stem cells, or myoblasts.
(15) The cell culture device according to any one of (1) to (14), wherein the stimulus-responsive region and the cell detachment inhibition region are produced by coating the cell culture surface with a temperature-responsive monomer, and then forming a stimulus-responsive layer by irradiating only the stimulus-responsive region with an electron beam, a γ-ray, or an ultraviolet ray.
(16) A kit for producing a cell sheet, comprising the cell culture device according to any one of (1) to (15) and a support for harvesting cell sheet to be used for cell sheet harvest.
(17) The kit for producing a cell sheet according to (16), wherein the support for harvesting cell sheet is paper, a cellulose film, or a plastic film having a thickness of 30 µm or more and 400 µm or less and a basis weight of 10 g/m² or more and 350 g/m² or less.
(18) The kit for producing a cell sheet according to (16) or (17), wherein the support for harvesting cell sheet has a smaller diameter or vertical and horizontal lengths as compared with the stimulus-responsive region.
(19) A method for producing a cell sheet, comprising a step of forming a cell sheet by culturing cells with the cell culture device according to any one of (1) to (15), and a step of harvesting the cell sheet with a support for harvesting cell sheet, wherein the support for harvesting cell sheet is paper, a cellulose film, or a plastic film having a thickness of 30 µm or more and 400 µm or less and a basis weight of 10 g/m² or more and 350 g/m² or less.
(20) A method for producing a cell sheet, comprising a step of culturing cells to form a cell sheet and then harvesting the cell sheet, by using the kit for producing a cell sheet according to any one of (16) to (18).
(21) A method of treatment using a cell sheet, comprising culturing cells using the cell culture device according to any one of (1) to (15), and
   harvesting the cells as a cell sheet using a support for harvesting cell sheet, wherein
   the support for harvesting cell sheet is paper, a cellulose film, or a plastic film having a thickness of 30 µm or more and 400 µm or less, and a basis weight of 10 g/m² or more and 350 g/m² or less, and
   the treatment is performed by placing the cell sheet with the support for harvesting cell sheet attached thereto on a cleaned affected area, and then removing the support for harvesting cell sheet.
(22) A method of treatment, comprising
   culturing cells using the cell culture device of the kit for producing a cell sheet according to any one of (16) to (18),
   collecting the cells as a cell sheet using a support for harvesting cell sheet,
   placing the cell sheet with the support for harvesting cell sheet attached thereto on a cleaned affected area, and then
   removing the support for harvesting cell sheet.
(23) The method of treatment according to (21) or (22), wherein a target to be treated is an extensive burn, a vitiligo, an ulcer, a pressure ulcer, a pulmonary fistula, an osteoarthritis, an esophageal stricture, a large or small intestinal stricture, an endometriosis, an osteoporosis, a renal fibrosis, a hepatic fibrosis, or an ischemic heart disease.

### Brief Description of Drawings

[FIG. 1] Fig. 1 schematically depicts examples of a stimulus-responsive region and a cell detachment inhibition region on the culture surface of the cell culture device of one embodiment.
[FIG. 2] Fig. 2 schematically depicts examples of a stimulus-responsive region and a cell detachment inhibition region on the culture surface of the cell culture device of another embodiment.
[FIG. 3] Fig. 3 depicts examples of a kit for producing a cell culture comprising a cell culture device and a support for harvesting cell sheet. Fig. 3(A) depicts a perspective view (left) of the culture device and a plan view (right) of the support for harvesting cell sheet.
Fig. 3(B) is a plan view of the culture device with the lid removed, and Fig. 3(C) depicts the support for harvesting cell sheet placed on the culture device.

### Description of Embodiments

The cell culture device described below is capable of conveniently harvesting a cell sheet without cell detachment until the cell sheet is harvested. Therefore, a cell sheet to be used for treatment in regenerative medicine, can be efficiently produced. Further, it can be suitably used as a cell culture device suitable for research and development in regenerative medicine or drug discovery research.

Any shape of the cell culture device included in the kit for producing a cell sheet may be used herein, and the device can be appropriately selected in consideration of the desired size and shape of a cell sheet. The cell culture device may have any shape as long as it is generally used for culturing cells, and is preferably in the shape of a vessel for usually culturing adherent cells, such as petri dishes, flasks, and multi-well plates. When a flask is used, and if it is a peel-off type flask or a flask with removable upper part, a cell sheet harvest by support for harvesting cell sheet can be easily performed since the upper part of the flask can be removed.

The present invention will be described below with reference to the drawings, but is not limited to the following embodiments. First, the cell culture device, which is a component of the kit for producing a cell sheet, will be described.

### [Embodiment 1]

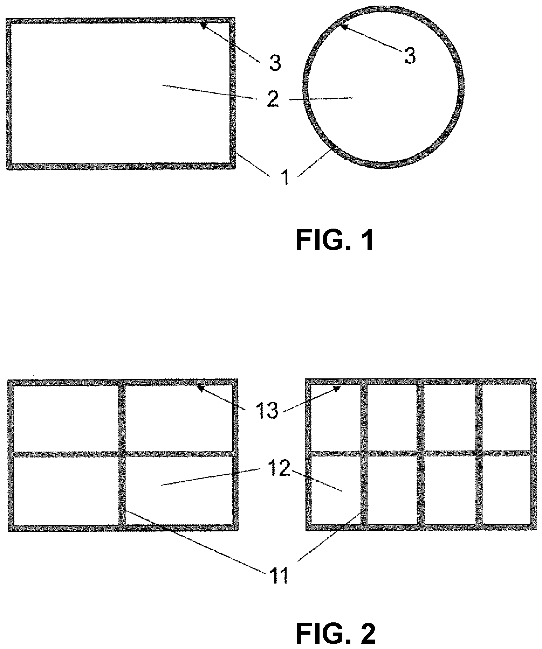

One embodiment of the cell culture device of the present invention is a cell culture device equipped with a cell detachment inhibition region 1 on the inner circumference of the culture device and a stimulus-responsive region 2 inside the cell detachment inhibition region 1. Fig. 1 is a diagram schematically depicting the culture surface of the cell culture device. Since the stimulus-responsive region 2 and the cell detachment inhibition region 1 are adjacent to each other, there is an adhesion/detachment boundary 3 which is the boundary portion. The culture surfaces of the cell culture devices illustrated in Fig. 1 are rectangular and circular ones, but may have any shape such as a square, and the optimum size can be appropriately selected depending on where the cell sheet is transplanted.

In the cell culture device, the stimulus-responsive region 2 is arranged in the center and the cell detachment inhibition region 1 is arranged on the outer edge thereof, wherein the cell detachment inhibition region 1 is directly provided over the entire circumference of the stimulus-responsive region 2 on the cell culture surface. Medium exchange is performed by aspirating the medium from a portion along the edge of the culture device, and then adding a medium. Therefore, if the cell adhesiveness of the outer edge of the culture surface is high, cell detachment is unlikely to occur due to mechanical stimulation upon medium exchange. Further, it is important that the cell detachment inhibition region is provided over the whole area along the inner circumference of the culture device. Since the cell detachment inhibition region is provided over the entire circumference, cell detachment can be prevented regardless of the position where the medium is aspirated or added. The cell detachment inhibition region 1 existing over the entire circumference allows the cells to firmly adhere to the surface of the culture device and avoid detachment due to mechanical stimulation upon medium exchange.

The cell detachment inhibition region 1 is a region composed of a surface that inhibits cell detachment within a temperature range of specifically 1°C or higher and 50°C or lower, regardless of the presence or absence of stimulation. It is desirable that the cell detachment inhibition region having high cell adhesiveness, but the degree of cell adhesiveness may be a degree such that the cell adhesiveness is maintained against mechanical stimulation such as aspiration and addition of a medium. Further, the adhesiveness to cells is preferably higher than the cell adhesiveness of the stimulus-responsive region in an unstimulated state.

For example, a culture device used in the following embodiments is processed so as to have a higher cell adhesion extension rate in a cell detachment inhibition region than that in a stimulus-responsive region, such that the cell adhesion extension rate in the stimulus-responsive region ranges from 5% to 20% of the culture surface, while the same in the cell detachment inhibition region ranges from 60% to 75% of the culture surface, when mouse neuroblastoma cell line Neuro2a is seeded at 5 × 10³ cells/cm² to 1 × 10⁵ cells/cm², and cultured for one hour using DMEM containing 10% FBS or FCS. Specifically, even in a state where no stimulation is applied to the stimulus-responsive region, the cell detachment inhibition region has cell adhesiveness 3 or more times and 15 or less times stronger than that of the stimulus-responsive region, when the cell adhesion extension rate is used as an index. This is because, although it depends on the size of the culture device, when the cell adhesion extension rate is used as an index, cells may be detached during culture unless the cell detachment inhibition region has adhesiveness 3 or more times stronger than that of the other. Further, in the case of a surface having adhesiveness more than 15 times stronger than that of the other, when cells are collected as a cell sheet, the edges of the cell sheet may not be detached and the cell sheet may be damaged. If the cell detachment inhibition region has cell adhesiveness 5 or more times stronger than that of the stimulus-responsive region, cell detachment during culture can be inhibited even when a culture device having a relatively large culture area is used. Further, if the cell detachment inhibition region has a surface having adhesiveness 9 or less times stronger than that of the stimulus-responsive region, even if cells have strong adhesiveness or the culture area is small, a cell sheet can be harvested without damage. It is known that cell adhesiveness differs depending on culture conditions such as the cell type, the culture period, and the medium used. Hence, through determination depending on the index of cell adhesiveness, size of the culture surface of the culture device, the one equipped with a cell detachment inhibition region having appropriate cell adhesiveness may be prepared.

The cell detachment inhibition region may be a surface with improved cell adhesion and proliferation. Therefore, it may be made of glass, which is generally used as a culture device, and surface-treated plastic. Examples of the plastic include polystyrene, polypropylene, polyvinyl chloride, and polycarbonate. Further, examples of the surface treatment that can be employed include methods usually used for reforming plastics such as corona discharge treatment, plasma treatment, UV ozone treatment, electron beam irradiation treatment, and laser treatment.

Further, in order to prevent cell detachment more positively, substances that physicochemically enhance cell adhesiveness, for example, basic polymers such as hydrophilic polystyrene and polylysine, basic compounds such as aminopropyltriethoxysilane, and N-(2-aminoethyl)-3-aminepropyltrimethoxysilane, and condensates containing these may be applied to the surface. Furthermore, substances that enhance cell adhesiveness because of the biochemical properties, such as fibrin, fibronectin, laminin, tenascin, hydronectin, collagen, atelocollagen, gelatin, RGD (arginine-glycine-aspartic acid) sequence-containing peptide, YIGSR (tyrosine-isoleucine-glycine-serine-arginine) sequence-containing peptide, and mixtures thereof may also be applied.

The stimulus-responsive region 2 may be a region where cell adhesiveness is varied by stimulation, and a temperature-responsive polymer having an upper or lower critical melting temperature of 0°C to 80°C can be preferably used. A preferred temperature-responsive polymer exhibits non-adhesiveness to cells at or higher than the upper critical melting temperature or at or lower than the lower critical melting temperature because it has high affinity for ambient water and takes up water to expand, and exhibits adhesiveness to cells at or below the upper critical melting temperature or at or above the lower critical melting temperature because of elimination of water from the polymer. In particular, the upper critical melting temperature is preferably 37°C or higher, at which cells are usually cultured, and the lower critical melting temperature (T) is preferably 37°C or lower, at which cells are usually cultured. Specifically, it is preferable that a surface of the culture device is a polymer that exhibits non-adhesiveness to cells in a temperature range of 0°C or higher and 32°C or lower.

Examples of such a temperature-responsive polymer include acrylic polymers or methacrylic polymers, and more specifically, poly-N-isopropylacrylamide (T = 32°C), poly-N-n-propylacrylamide (T = 21°C), poly-N-n-propylmethacrylamide (T = 32°C), poly-N-ethoxyethylacrylamide (T = about 35°C), poly-N-tetrahydrofurfurylacrylamide (T = about 28°C), poly-N-tetrahydrofurfurylmethacrylamide (T = about 35°C), and poly-N,N-diethylacrylamide (T = 32°C).

Examples of monomers for forming these polymers include monomers that can be polymerized by irradiation with radiation such as γ-rays, electron beams, ultraviolet rays, etc. For example, a (meth)acrylamide compound, an N-alkyl substituted (meth)acrylamide derivative, an N,N-dialkyl-substituted (meth)acrylamide derivative, a (meth)acrylamide derivative having a cyclic group, a vinyl ether derivative and the like may be used alone or two or more types thereof may be mixed and then used in combination.

By coating a cell culture device with the above polymerizable monomers and then irradiating the device with an electron beam or radiation, the monomers are polymerized to form a polymer layer and then a stimulus-responsive region is formed. Examples of a method for forming the cell detachment inhibition region include a method comprising forming a stimulus-responsive region over the entire surface of the culture area and then removing the stimulus-responsive layer, and a method wherein a cell detachment inhibition region is shielded from an electron beam or the like to avoid polymerization, thereby partially preventing the formation of a stimulus-responsive layer. Here, the portion where the cell detachment inhibition region is desired to be formed is shielded so that the polymer is not formed. When the stimulus-responsive region is removed and the cell detachment inhibition region is formed, it is necessary not only to grasp the thickness of the layer on which the polymer is formed and completely remove it, but also to manage, such as to eliminate the removed polymer from the culture surface. If a method of shielding the cell detachment inhibition region from irradiation with an electron beam or the like is used, there is no need to remove the stimulus-responsive layer, and the cell detachment inhibition region and the stimulus-responsive region can be formed by a very simple step.

The width of the cell detachment inhibition region 1 is preferably 2 mm or more and 10 mm or less. The mechanical stimulation is applied by aspiration and addition of a medium from the wall of the culture device to a predetermined width of the region without depending on the size of a culture device or the like. If the width of the cell detachment inhibition region is narrower than 2 mm, sufficient cell adhesive force cannot be obtained, and the cell sheet may be detached. Therefore, if the cell detachment inhibition region is narrow, cell detachment cannot be prevented. Further, if the width is wide, stable adhesion force can be obtained, but the reduce the area available for cell collection. Further, if the width is wide, cells are not detached from the culture device, and are harvest cell sheet with difficulty from the culture device. Therefore, as a result of ensuring a sufficiently stable adhesion force and examining the harvesting efficiency of a cell sheet, the width of the cell detachment inhibition region is preferably 2 mm or more and 10 mm or less, and more preferably 4 mm or more and 8 mm or less. As described above, the cell detachment inhibition region may have cell adhesive force stronger than that of the stimulus-responsive region at the cell culture temperature, but is required to maintain its cell adhesive force at a level regardless of the presence or absence of stimuli to detach the cells.

As described above, when a cell culture device having a large culture area is used, detachment tends to take place upon medium exchange. With the actual use of primary cultured cells of sheep keratinocytes, whether the ease of detachment differs depending on the culture area was examined. Specifically, this was examined using a cell culture device having only a stimulus-responsive region or a cell culture device having a cell detachment inhibition region with a width of 5 mm disposed around a stimulus-responsive region. Regardless of which culture device was used, the examination was carried out by adjusting the number of cells to be seeded according to the area, in the schedule of medium exchange on day 4 and day 7 and cell sheet recovery on day 10. After the start of cell culture, cells proliferated to reach semiconfluence in all the culture devices on day 7 and to reach confluence on day 10. The material of all plates was polystyrene. Stimulus-responsive region was formed through coating with a temperature-responsive polymer and the same temperature-responsive surface treatment.

**[Table 1]**

| Culture device | 12 well plate | Φ 3.5 cm dish | Φ 10 cm dish | Single-well plate |
|---|---|---|---|---|
| Culture area (cm²) | 3.5 | 8.8 | 56.7 | 84 |
| Cell culture device equipped with only stimulus-responsive region | Δ | X | X | X |
| | On day 7, detached in 3 out of 12 wells. | On day 7, detached during medium exchange. N=3 | On day 7, detached during medium exchange. | On day 7, detached during medium exchange. N=3 |
| Cell culture device equipped with cell detachment inhibition region and stimulus-responsive region | - | O | - | O |
| | - | Cell sheet was harvested on day 10 without detachment during culture period. N=3 | - | Cell sheet was harvested on day 10 without detachment during culture period. N=3 |

As shown in Table 1, even with the culture area of 3.5 cm², cell detachment was identified at a high frequency of 3 out of 12 wells in the culture devices having no cell detachment inhibition region. Further, with the culture area of larger than 8.8 cm², cells were detached during medium exchange on day 7 and could not be recovered as a cell sheet. In the case of a cell culture device equipped with a cell detachment inhibition region and a stimulus-responsive region, cells were successfully harvested without problems even with 8.8 cm² culture devices, suggesting that cells can be collected as a cell sheet without any problem although a case of a culture area of 3.5 cm² was not confirmed. Further, a comparative study was conducted herein using a plate having a culture area of 84 cm², confirming that the cell sheet can be harvested without any problem even with the culture area of 225 cm². The above results indicate that the cell detachment inhibition region having cell adhesiveness stronger than that of the stimulus-responsive region is required.

Further, the cell detachment inhibition region and the stimulus-responsive region are arranged on the same plane. This is because if the cell detachment inhibition region and the stimulus-responsive region are uneven, there will be a portion that does not adhere to an affected area when cells are collected as a cell sheet and the sheet is clinically applied to the affected area, and the engraftment property is considered to be inferior.

A cell sheet obtained by culturing various cells can be transplanted to an affected area for treatment. For example, an epidermal cell sheet can be used for treatment of extensive burns, vitiligos, ulcers, and pressure ulcers, a cartilage sheet can be used for treatment of osteoarthritis, a mesenchymal stem cell sheet can be used for treatment of brain diseases such as dementia, breast reconstruction, osteoarthritis/traumatic cartilage defect, and ischemic heart disease, a fibroblast sheet can be used for treatment of pulmonary fistula, an oral mucosal epithelial cell sheet can be used for treatment of esophageal strictures, large or small intestinal strictures, endometriosis, and an osteoblast sheet can be used for treatment of osteoporosis, a renal cell sheet can be used for treatment of renal fibrosis, a hepatocellular sheet can be used for treatment of liver fibrosis, and myoblast, myocardial cell, and smooth muscle cell sheets can be used for treatment of ischemic heart disease. Note that cells that can be used as a cell sheet are not limited to the above, and diseases to which the cell sheet is applied may be any disease as long as the disease is the one for which improvement is confirmed by cell transplantation. Cells to be used herein may be either autologous or allogeneic cells. Examples of cell types include skin keratinocytes, pigment cells (melanocytes), skin fibroblasts (fibroblasts), sebaceous gland cells (sebocytes), granule cells, prickle cells, basal cells, Langerhans cells, and Merkel cells. Moreover, stem cells including induced pluripotent stem cells, embryonic stem cells, or somatic stem cells may be used by inducing differentiation. When skin is collected from a patient and cells are cultured, a culture period of about 3 weeks is required. Accordingly, if there is a stock prepared by induction of differentiation of induced pluripotent stem cells, etc., such a stock can be immediately used as needed, and is useful for treatment of patients with severe burns.

Treatment using a cell sheet is performed as follows, for example. The most successful treatments using cell sheets are those using epithelial tissue-derived cell sheets for burns, vitiligo vulgaris, ulcers, and pressure ulcers. In the treatment using a cell sheet, cells derived from an epithelial tissue such as an oral mucosal tissue or a skin tissue are separated from the tissue, and the treatment is performed using each type of the obtained cells and a cell population containing two or more types thereof.

First, cells are collected from a patient or another person's tissue fragment and cultured. The cells are cultured in a cell culture device having a stimulus-responsive region and a cell detachment inhibition region to prepare for cell sheet preparation. Once the cells are ready, debridement that is a surgical procedure to remove the affected epidermis and infected/necrotic tissue and clean the wound. The cells cultured to confluence are collected as a cell sheet using a support for harvesting cell sheet. The cell sheet including the support for harvesting cell sheet attached thereto is placed on an affected area, so that the cell sheet side is in contact with the affected area. Therefore, a side of the cell sheet, which is adhered to the cell culture device, that is, the side having high adhesiveness to the scaffold directly covers the affected area. After few minutes to 10 minutes, when only the support is slowly removed, only the cell sheet engrafts on the affected area. When an affected area including dermis has been removed, the cell sheet is overlaid on a portion where artificial dermis has been transplanted for transplantation. Further, as in the case of patch skin grafting of skin grafts, thrombin solution/fibrinogen can also be used for engraftment and fixation.

To date, there has been no culture device with a cell detachment inhibition region having predetermined cell adhesiveness and width provided around a stimulus-responsive region in order to prevent cells from being detached during cell culture. For transplantation, it is necessary to culture cells until they reach confluence. Provision with the cell detachment inhibition region makes it possible to culture cells without detachment during the cell culture period.

### [Embodiment 2]

Next, a culture device in which one culture surface is subdivided will be described. Fig. 2 schematically depicts the culture surfaces of the culture devices in which each culture surface is subdivided. Here, examples are depicted in which the culture surface of the culture device is divided into four (left in FIG. 2) or eight (right in FIG. 2) by the cell detachment inhibition region 11. Here, an example in which a rectangular culture device is subdivided is shown, but any shape of the culture device such as a square or a circular culture device can be similarly subdivided by the cell detachment inhibition region 11. Further, depending on the intended use, an area to be divided may be divided into large and small sections instead of being divided into sections of the same size. In this form, the cell detachment inhibition region 11 is also present inside the culture surface along the outer circumference of the stimulus-responsive region 12. The adhesion/detachment boundary 13 exists as the outer circumference of each stimulus-responsive region 12 subdivided by the cell detachment inhibition region 11.

Even in the case of a culture device having a subdivided culture surface, the width of the cell detachment inhibition region 11 is preferably set to 2 mm or more and 10 mm or less, and more preferably 4 mm or more and 8 mm or less. The use of a culture device equipped with subdivided sections makes it possible not only to conduct different analysis for each section as described above, but also to harvest a cell sheet of each section and to apply the thus harvested cell sheets to different affected areas. Furthermore, since the cell detachment inhibition region exists not only on the outer circumference of the stimulus-responsive region but also inside the region, making it possible to inhibit cell detachment more strongly.

Since a surface of the culture device of the present embodiment is subdivided by the cell detachment inhibition region, making it possible to analyze cells cultured under the same culture conditions by different analysis methods. By using cells cultured in the same culture device, more accurate analysis results can be obtained. Further, according to the present embodiment, it is also possible to apply a cell sheet cultured in one culture device to a plurality of affected areas.

### [Embodiment 3]

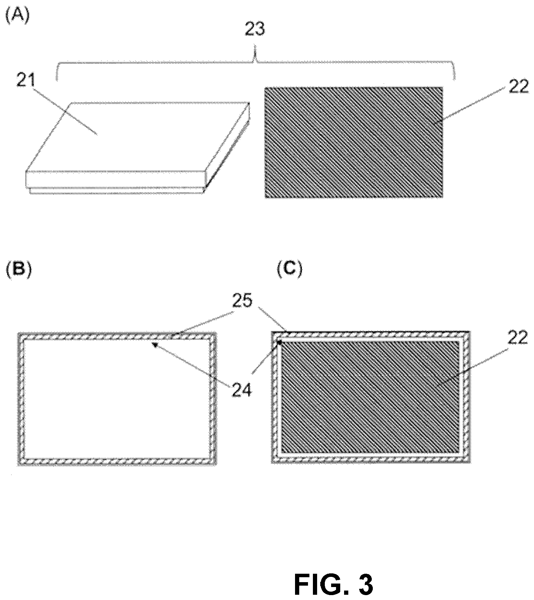

Next, a kit for producing a cell sheet 23 comprising a culture device 21 and a support for harvesting cell sheet 22 for harvesting a cell sheet will be described (Fig. 3A). In order to collect the cultured cells in the form of a sheet, it is preferable to use the support for harvesting cell sheet 22. The cells are adhered to the support for harvesting cell sheet 22, so that the resultant can be easily held with tweezers or the like.

The cell sheet is harvested as follows. After cells reach confluence, the medium is removed by aspiration. After aspirating the medium, a small amount of medium is added to prevent the cells from drying out during the operation. The amount of the medium may be about 5 µl/cm² to 30 µl/cm². Next, the support for harvesting cell sheet 22 is placed on the cells. The support for harvesting cell sheet 22 is one size smaller than the stimulus-responsive region. For example, in the case of a rectangular or square-shaped culture device, the support for harvesting cell sheet 22 may have a length and a width shorter than those of the stimulus-responsive region by about 1 mm to 5 mm, respectively. In the case of a circular shape, the support for harvesting cell sheet 22 having a diameter shorter than that of the stimulus-responsive region by about 1 mm to 5 mm may be prepared.

For example, when the stimulus-responsive region is temperature-responsive and the lower critical melting temperature is 32°C, a cell sheet is allowed to stand at a temperature of 32°C or lower, preferably 0°C to 30°C, for about 5 to 60 minutes. After standing still, when the support for harvesting cell sheet is held and lifted with tweezers or the like, the cells adhere to the support for harvesting cell sheet in the form of a sheet and are detached from the cell culture device. In the case of cells having high adhesiveness to the cell detachment inhibition region, the periphery of the support for harvesting cell sheet may be detached with tweezers or the like. The cell sheet adhered to the support for harvesting cell sheet may be placed as it is on a site to which the cell sheet is transplanted. The side opposite to the side adhering to the support for harvesting cell sheet, that is, the exposed side is the side adhering to the surface of the culture device. That is, it is the side that has adhered to the scaffold, and this side can be brought into close contact with a side to which transplantation should be performed.

Further, the support for harvesting cell sheet 22 is desirably placed at a distance of 1 mm to 2 mm from and inside the adhesion/detachment boundary 24 formed by the stimulus-responsive region and the cell detachment inhibition region, although this depends on its size (Fig. 3(C)). This is because cells are not detached due to stimulation in the cell detachment inhibition region, and therefore, if cells are firmly adhered to the surface of the device, there is a risk that the cell sheet may be torn. The support for harvesting cell sheet is prepared to be one size smaller than the stimulus-responsive region, but it is very difficult to place the support for harvesting cell sheet inside the adhesion/detachment boundary. In particular, when the culture surface is large, it is a fine procedure to place it inside both vertically and horizontally.

As depicted in Fig. 3(B) and Fig. 3(C), a boundary display mark 25 that can be visually confirmed is provided in a region corresponding to the cell detachment inhibition region, so that the adhesion/detachment boundary 24 can be identified. Since the device surface of the stimulus-responsive region and the cell detachment inhibition region is not an optically changed surface, the location of the adhesion/detachment boundary is unrecognizable. However, since a position where the support for harvesting cell sheet 22 should be placed can be visually recognized with the boundary display mark 25, the support for harvesting cell sheet can be reliably placed inside the adhesion/detachment boundary 24.

The boundary display mark 25 may be formed by frosting or lightly coloring a surface or the bottom surface of the culture device at a position corresponding to the cell detachment inhibition region or the adhesion/detachment boundary. The light-transmitting process does not cause any problem when the proliferation and morphology of cells are observed under a microscope. Moreover, cell proliferation and the like can be confirmed in the stimulus-responsive region, and even if the cell detachment inhibition region is processed or colored, there is no problem at all for culturing. The side to be processed may be a side to which cells adhere, or may be the outside of the device as long as the processing does not affect cell adhesion.

### [Examination of support for harvesting cell sheet]

Since the harvest of cell sheets is delicate operation, it is important to have a support for harvesting cell sheet that is easy to operate. For the support for harvesting cell sheet 22, known materials such as paper, a cellulose film, and a plastic film can be used. It is preferable to use paper, a cellulose film, or a plastic film because of its good adhesiveness to cells. However, the materials are preferably materials that do not tear even when immersed in a culture solution and are flexible and easy to handle since they are used in delicate operation. Using the support for harvesting cell sheet having different thicknesses and basis weights, the prepared cell sheets were transferred to examine the optimum range of the support for harvesting cell sheet.

Cell sheets were prepared under the following conditions. Human adipose tissue-derived mesenchymal stem cells (human Adipose-derived stem cell, hADSC) and media were purchased from LONZA. Cells used herein were previously cryopreserved in a human adipose-derived stem cell medium (ADSC-BulletKit^{™}) after two passages. After thawing the cryopreserved hADSC, cells were suspended in a human adipose-derived stem cell medium (ADSC-Bullet Kit^{™}) supplemented with an ascorbic acid derivative and FGF-2, thereby preparing a 1.5 × 10⁵ cells/mL cell suspension. Two mL of the thus prepared cell suspension was seeded on a dish as a temperature-responsive culture device having a diameter of 3.5 cm and equipped with a cell detachment inhibition region, followed by 5 days of culture at 37°C. During the culture period, the medium was exchanged once on day 4. The cells reached confluence on day 5. Examination was conducted using supports having the following thicknesses and basis weights.

**[Table 2]**

| | Thickness (µm) | Basis weight (g/m²) |
|---|---|---|
| Example 1 | 40 | 32 |
| Example 2 | 240 | 240 |
| Comparative Example 1 | 630 | 435 |

After it was confirmed that the hADSC cells had been cultured to reach confluence, the medium was removed by aspiration. Next, the support for harvesting cell sheet of Examples 1 and 2 and Comparative Example 1 having the same circular shape as that of the culture surface of the culture device were pre-moistened with a medium and placed on the confluent cells. The cells were left to stand at 20°C for 10 minutes, one end of the support for harvesting cell sheet was held at one point with tweezers, and then the support for harvesting cell sheet was slowly lifted upward while bending it. When the supports for harvesting cell sheet of Examples 1 and 2 were used, cell sheets attached to the support for harvesting cell sheet could be harvested. Furthermore, it was possible to remove only the support for harvesting cell sheet after the transfer of the cell sheets. On the other hand, when the support for harvesting cell sheet of Comparative Example 1 was used, one end of the support for harvesting cell sheet was held with tweezers at one point, so as to try to recover the cell sheet. However, the support for harvesting cell sheet was difficult to be rolled up, and thus lifted as it was. As a result, the cell sheet was recovered with a portion cut off. From these results, it was concluded that the upper limits of a support for harvesting cell sheet are 400 µm or less in thickness and 350 g/m² or less in basis weight. Further, when the lower limits of the support for harvesting cell sheet were examined, paper having a basis weight of less than 10 g/m² was very difficult to handle such that the edge was cut off when the wet paper was held with tweezers. Since the basis weight and the thickness are related numerical values, it is considered that the lower limits of the support for harvesting cell sheet are 30 µm in thickness and 10 g/m² in basis weight. It was concluded that the use of the one having a thickness and a basis weight larger than such values is preferable.

From the above results, it is preferable that the support for harvesting cell sheet has a thickness of 30 µm or more and 400 µm or less, and further preferably a thickness of 30 µm or more and 300 µm or less. Further, the basis weight is preferably 10 g/m² or more and 350 g/m² or less, and further preferably 20 g/m² or more and 250 g/m² or less. With a thickness and a basis weight within these ranges, the support for harvesting cell sheet is easily held with tweezers, facilitating delicate operations. Further, regarding the properties of the material, a flexible and soft material is preferable. As described above, the material is preferably paper, a cellulose film, or a plastic film, and specific examples of the material that can be preferably used herein include hydrated PTFE (polytetrafluoroethylene), a PGA (polyglycolic acid) resin film, a PLA (polylactic acid) resin film, a PLGA (polylactic acid/glycolic acid copolymer) resin film, sulfate paper, a cellulose film, and sterilized paper.

The kit for producing a cell sheet of the present embodiment inhibits cell detachment during the cell culture period, and is capable of easily harvesting a cell sheet when the cell sheet is harvested. The kit for producing a cell sheet equipped with a culture device and a support for harvesting cell sheet has an unprecedented configuration. Using the kit for producing a cell sheet, the produced cell sheet can be simply held in the form of a sheet. Therefore, handling of cell sheets used to require some training, but even beginners can now handle them.

### Reference Signs List

1, 11...cell detachment inhibition region, 2, 12...stimulus-responsive region, 3, 13, 24... adhesion/detachment boundary, 21...culture device, 22...support for harvesting cell sheet, 23...kit for producing a cell sheet, 25...boundary display mark

## Claims

1. A cell culture device, wherein
a cell detachment inhibition region having cell adhesive force stronger than that of a stimulus-responsive region regardless of the presence or absence of a stimulus and inhibiting cell detachment is provided over the entire circumference of a cell culture surface,
the stimulus-responsive region having adhesiveness to cells, which is varied by an external stimulus, is arranged inside the cell detachment inhibition region, and
the cell detachment inhibition region has cell adhesiveness 3 or more times and 15 or less times stronger than that of the stimulus-responsive region even in a state where no stimulation is applied to the stimulus-responsive region.

2. The cell culture device according to claim 1,
wherein
the container shape of the cell culture device is a petri dish, a flask, or a multi-well plate, and the stimulus-responsive region and the cell detachment inhibition region are directly provided on the cell culture surface of the cell culture device.

3. The cell culture device according to claim 1 or 2, which is for the harvesting of a cell sheet using a support for harvesting cell sheet.

4. The cell culture device according to any one of claims 1 to 3, wherein the cell culture surface of the cell culture device has an area of 3.5 cm² or more and 300 cm² or less.

5. The cell culture device according to any one of claims 1 to 4, wherein the cell detachment inhibition region is a cell adhesive surface in a temperature range of 1°C or higher and 50°C or lower.

6. The cell culture device according to claim 5,
wherein the cell adhesive surface is a culture surface not responding to an external stimulus.

7. The cell culture device according to any one of claims 1 to 6, wherein the external stimulus is light, a temperature, a pH, or a magnetic field.

8. The cell culture device according to any one of claims 1 to 7, wherein the stimulus-responsive region is a temperature-responsive surface responding to temperature stimulation, and a surface from which cells are detached at a temperature of 0°C or higher and 32°C or lower.

9. The cell culture device according to any one of claims 1 to 8, wherein the cell detachment inhibition region and the stimulus-responsive region are distinct sections on the same plane.

10. The cell culture device according to any one of claims 1 to 9, wherein the cell detachment inhibition region has a width of 2 mm or more and 10 mm or less.

11. The cell culture device according to any one of claims 1 to 10, wherein the cell detachment inhibition region is provided so as to divide the cell culture surface in addition to the periphery of the cell culture surface.

12. The cell culture device according to any one of claims 1 to 11, wherein the cell detachment inhibition region and the stimulus-responsive region are processed so as to be visually distinguishable.

13. The cell culture device according to any one of claims 1 to 12, wherein the cell detachment inhibition region and the stimulus-responsive region are optically transparent.

14. The cell culture device according to any one of claims 1 to 13, which is for producing a cell sheet of epithelial cells, mesenchymal stem cells, or myoblasts.

15. The cell culture device according to any one of claims 1 to 14, wherein the stimulus-responsive region and the cell detachment inhibition region are produced by coating the cell culture surface with a temperature-responsive monomer, and then forming a stimulus-responsive layer by irradiating only the stimulus-responsive region with an electron beam, a γ-ray, or an ultraviolet ray.

16. A kit for producing a cell sheet, comprising the cell culture device according to any one of claims 1 to 15 and a support for harvesting cell sheet to be used for cell sheet harvest.

17. The kit for producing a cell sheet according to claim 16, wherein the support for harvesting cell sheet is paper, a cellulose film, or a plastic film having a thickness of 30 µm or more and 400 µm or less and a basis weight of 10 g/m² or more and 350 g/m² or less.

18. The kit for producing a cell sheet according to claim 16 or 17, wherein the support for harvesting cell sheet has a smaller diameter or vertical and horizontal lengths as compared with the stimulus-responsive region.

19. A method for producing a cell sheet, comprising a step of forming a cell sheet by culturing cells with the cell culture device according to any one of claims 1 to 15, and a step of harvesting the cell sheet with a support for harvesting cell sheet, wherein the support for harvesting cell sheet is paper, a cellulose film, or a plastic film having a thickness of 30 µm or more and 400 µm or less and a basis weight of 10 g/m² or more and 350 g/m² or less.

20. A method for producing a cell sheet, comprising a step of culturing cells to form a cell sheet and then harvesting the cell sheet, by using the kit for producing a cell sheet according to any one of claims 16 to 18.
